# EUROPÄISCHE PATENTANMELDUNG

(11) **EP 1 860 234 A1**
(43) Veröffentlichungstag der Anmeldung: **28.11.2007**
(21) Anmeldenummer: 07105708.7
(22) Anmeldetag: 05.04.2007
(51) Int. Cl.: D21F 7/04, G01N 21/89, B41F 33/18, B65H 26/02, G01N 33/34

(54) **Verfahren und Vorrichtung zur Bahnabrissüberwachung einer Materialbahn**

(30) Priorität: 24.05.2006 DE 102006024344
(71) Anmelder: Voith Patent GmbH, 89522 Heidenheim (DE)
(72) Erfinder: Hennes, Patrick, 89542 Bolheim (DE); Buttschardt, Werner, 89542, Herbrechtingen (DE)
(74) Vertreter: Kunze, Klaus

(57) **Zusammenfassung**

Die Anmeldung betrifft ein Verfahren zur Bahnabrissüberwachung in einer der Herstellung und/oder Behandlung einer Materialbahn, insbesondere einer Papier- oder Kartonbahn, dienenden Maschine, insbesondere Papier- oder Streichmaschine, in welcher die Materialbahn mit einer vorgegebenen Geschwindigkeit in Längsrichtung entlang einer vorgegebenen Wegstrecke geführt wird, wobei ein Bahnabriss mittels eines lichtemittierenden Bauteils und eines lichtempfangenden Sensors erfasst wird, indem Licht von dem lichtemittierenden Bauteil in Richtung der Materialbahn zu einem vorgegebenen Zielpunkt gestrahlt wird, in Abhängigkeit des Vorhandenseins der Materialbahn in dem Zielpunkt reflektiert und/oder durchgelassen und/oder zu dem lichtempfangenden Sensor geleitet wird.

Die Anmeldung ist dadurch gekennzeichnet, dass das Licht derart von einer Vielzahl von nebeneinander auf einer ersten Seite der Materialbahn (1) angeordneten lichtemittierenden Bauteilen (2) oder von einem lichtemittierenden Bauteil mit einer linienförmigen Lichtquelle auf der ersten Seite in Richtung einer zugeordneten Vielzahl von nebeneinander angeordneten lichtempfangenden Sensoren (3) auf einer zweiten, der ersten Seite entgegengesetzt angeordneten Seite der Materialbahn gestrahlt wird, dass ein Lichtgitter (4) ausgebildet wird, welches sich in Querrichtung der Materialbahn über die gesamte Breite derselben erstreckt.

## Beschreibung

Die Erfindung betrifft ein Verfahren und eine Vorrichtung zur Bahnabrissüberwachung in einer der Herstellung und/oder Behandlung einer Materialbahn, insbesondere einer Papier- oder Kartonbahn, dienenden Maschine, insbesondere Papier- oder Streichmaschine, in welcher die Materialbahn mit einer vorgegebenen, in der Regel konstanten, Geschwindigkeit in ihrer Längsrichtung entlang einer vorgegebenen Wegstrecke geführt wird.

Verfahren und Vorrichtungen zur Bahnabrissüberwachung sind in verschiedenen Ausführungen bekannt. Beispielsweise wird auf die Offenlegungsschriften DE 101 57 914 A1 und DE 101 57 915 A1 verwiesen, in welchen vorgeschlagen wird, das Abreißen einer Papierbahn durch Erfassen der Bahngeschwindigkeit oder des Bahnzugs zu bestimmen.

Ferner beschreibt die Offenlegungsschrift DE 42 16 653 A1 eine Vorrichtung zum Durchtrennen einer Papierbahn in einer Pressenpartie einer Papiermaschine, bei welcher ein Abrissdetektor mit einer Sende-/Empfangseinheit vorgesehen ist, der über einen Vergleich der Farbtönung des Trockenfilzes des aktuellen Abrisses und eines vorhergehenden Messwertes eine Abschlagvorrichtung dann aktiviert, wenn der Farbvergleich zu einer Übereinstimmung führt.

Schließlich zeigt das US-Patent 5 289 007 eine Erkennungsvorrichtung für einen Bahnabriss, welche mit auf entgegengesetzten Seiten der Bahn angeordneten Bauteilen, nämlich einem Infrarotsender und einem Infrarotempfänger, arbeitet.

Die Verfahren und Vorrichtungen gemäß dem Stand der Technik arbeiten entweder mit von Sensoren erfasster reflektierter Strahlung oder nur punktuell. Dabei kommen zum einen komplizierte und störungsanfällige Sensoren zum Einsatz, die in bestimmten Anwendungsfällen, insbesondere bei sehr hohen Bahngeschwindigkeiten, nicht immer zuverlässig arbeiten. Zum anderen hat sich in der Praxis herausgestellt, dass ein Bahnabriss häufig nur dann zuverlässig und ausreichend schnell erkannt werden konnte, wenn ein Totalabriss der Bahn stattgefunden hat.

Der Erfindung liegt die Aufgabe zugrunde, ein Verfahren und eine Vorrichtung zur Bahnabrissüberwachung anzugeben, welche gegenüber dem Stand der Technik verbessert ist und insbesondere zu einer sichereren und schnelleren Erkennung von Bahnabrissen, auch Teilabrissen, sowie vorteilhaft von strukturellen Oberflächenfehlern führt.

Die erfindungsgemäße Aufgabe wird durch ein Verfahren mit den Merkmalen von Anspruch 1 und eine Vorrichtung gemäß dem Anspruch 9 gelöst. Die abhängigen Ansprüche beschreiben vorteilhafte und besonders zweckmäßige Ausgestaltungen der Erfindung.

Gemäß der Erfindung ist es möglich, die Materialbahn, insbesondere Papierbahn, über der gesamten Maschinenbreite zuverlässig und insbesondere unabhängig von der Bahngeschwindigkeit zu überwachen. Ein Abriss, auch Teilabriss, kann somit schneller erkannt werden, als dies beispielsweise bei einer punktuellen Überwachung der Papierbahn möglich ist. Auch wenn sich die Materialbahn der Länge nach teilt, kann dies durch die erfindungsgemäße Ausgestaltung zuverlässig erfasst werden.

Schließlich können auch einzelne Löcher in der Bahn und insbesondere das Durchhängen der Bahn erkannt werden und somit im Idealfall festgestellt werden, dass ein Abriss, der zwar noch nicht begonnen hat, demnächst bevorsteht.

Der Grundgedanke der Erfindung lässt sich darin zusammenfassen, dass nicht ein einzelner Punkt der Materialbahn überwacht wird, sondern dass ein Lichtgitter auf die beziehungsweise gemäß einer Ausführung der Erfindung stets durch die Materialbahn gestrahlt wird, dass durch Vorsehen einer entsprechenden Anzahl von nebeneinander angeordneten lichtempfangenden Sensoren erfasst wird und eine Bewertung des Zustandes der Materialbahn über ihrer gesamten Bahnbreite ermöglicht.

Wenn die Intensität des von den lichtemittierenden Bauteilen in Abhängigkeit der Lichtdurchlässigkeit der Materialbahn derart eingestellt wird, dass die zugeordneten lichtempfangenden Sensoren stets das ausgestrahlte Licht empfangen, so kann anhand der Intensität des empfangenen Lichtes festgestellt werden, ob die Materialbahn vorhanden ist beziehungsweise welche Dichte die Materialbahn an dem Zielpunkt, an welchem der Lichtstrahl des entsprechenden lichtemittierenden Bauteils die Materialbahn durchdringt, aufweist. Auch ist es möglich, festzustellen, ob der Lichtstrahl mehrfach durch die Materialbahn hindurchgestrahlt wurde. Bei der erfindungsgemäßen gegenüberstehenden Anordnung von Sendern und Empfängern des einzelnen Lichtstrahls auf entgegengesetzten Seiten der Materialbahn ist ein doppelter Durchgang nur dann möglich, wenn die Materialbahn in Richtung ihrer Längsachse beziehungsweise bei im spitzen Winkel zur Längsachse der Materialbahn durchgestrahlten Lichtstrahlen auch in Richtung ihrer Querachse durchhängt. Ein solches Durchhängen ist unerwünscht, und die Erfindung ermöglicht, durch Erfassen des Durchhängens geeignete Maßnahmen zu ergreifen.

Wenn die Intensität des empfangenen Lichtstrahls beispielsweise einen vorgegebenen Grenzwert überschreitet, so steht fest, dass entweder keine Materialbahn in dem entsprechenden Zielpunkt vorhanden war oder die Dichte beziehungsweise das Flächengewicht der Materialbahn zu gering ist. Eine zu geringe Dichte kann Keimstelle für einen beginnenden Abriss sein. Somit können erfindungsgemäß Abrisse bereits erkannt werden, bevor sie in Form von tatsächlichen Öffnungen oder Risslinien auftreten.

Im einzelnen umfasst das erfindungsgemäße Verfahren zur Bahnabrissüberwachung in einer der Herstellung und/oder Behandlung einer Materialbahn, insbesondere einer Papier- oder Kartonbahn, dienenden Maschine, insbesondere Papier- oder Streichmaschine, in welcher die Materialbahn mit einer vorgegebenen Geschwindigkeit in ihrer Längsrichtung entlang einer vorgegebenen Wegstrecke geführt wird, und wobei ein Bahnabriss mittels eines lichtemittierenden Bauteils und eines lichtempfangenden Sensors erfasst wird, indem Licht von dem lichtemittierenden Bauteil in Richtung der Materialbahn zu einem vorgegebenen Zielpunkt und in Abhängigkeit des Vorhandenseins der Materialbahn in dem Zielpunkt reflektiert und/oder durchgelassen und/oder zu dem lichtempfangenden Sensor geleitet wird, das Ausstrahlen von Licht entweder mit einer Vielzahl von nebeneinander auf einer ersten Seite der Materialbahn angeordneten lichtemittierenden Bauteilen oder mit einem Bauteil auf der ersten Seite, das eine linienförmige Lichtquelle umfasst, in Richtung einer zugeordneten Vielzahl von nebeneinander angeordneten lichtempfangenden Sensoren. Die lichtempfangenden Sensoren sind dabei auf einer zweiten Seite der Materialbahn angeordnet, welche zweite Seite der ersten Seite entgegengesetzt positioniert ist. In anderen Worten, die lichtemittierenden Bauteile und die lichtempfangenden Sensoren sind auf verschiedenen Seiten der Materialbahn angeordnet, so dass sie die Materialbahn zwischen sich einschließen.

Mittels des lichtemittierenden Bauteils beziehungsweise der Vielzahl von nebeneinander angeordneten lichtemittierenden Bauteilen wird ein Lichtgitter in Richtung der zugeordneten Vielzahl von lichtempfangenden Sensoren ausgestrahlt, das sich in Querrichtung der Materialbahn vollständig über deren gesamte Breite erstreckt. Unter gesamte Breite im Sinne der vorliegenden Erfindung ist dabei die gesamte Erstreckung von dem ersten seitlichen Rand der Materialbahn bis zu ihrem zweiten seitlichen Rand zu verstehen. Insbesondere ist es jedoch unschädlich, wenn einzelne Bereiche der Materialbahn, insbesondere an dessen äußersten seitlichen Rändern, nicht vollständig von dem Lichtgitter abgedeckt werden. Wichtig ist, dass im wesentlichen die gesamte Breite der Materialbahn in Querrichtung abgedeckt wird. Gemäß einer vorteilhaften Ausführung der Erfindung erstreckt sich das Lichtgitter jedoch kontinuierlich über die gesamte Breite der Materialbahn in Querrichtung.

Wenn die Materialbahn waagerecht geführt wird, kann beispielsweise das lichtemittierende Bauteil beziehungsweise können die lichtemittierenden Bauteile oberhalb der Materialbahn und die lichtempfangenden Sensoren unterhalb der Materialbahn angeordnet sein, oder umgekehrt. In diesem Fall weisen das beziehungsweise die lichtemittierenden Bauteile und die lichtempfangenden Sensoren insbesondere jeweils mindestens oder genau die Breite der Materialbahn in Querrichtung, das heißt von seitlichem Rand zu seitlichem Rand, auf. Um die Breite der nebeneinander angeordneten lichtemittierenden Bauteile, welche vorteilhaft zu einer einzigen Senderschiene zusammengefügt beziehungsweise integriert sind, und der lichtempfangenden Sensoren, welche vorteilhaft ebenfalls zu einer einzigen Schiene, einer Empfängerschiene, zusammengefügt oder integriert sind, zu vermindern, sind die lichtemittierenden Bauteile und die lichtempfangenden Sensoren jedoch vorteilhaft derart angeordnet, dass das Lichtgitter in einem spitzen Winkel auf eine der beiden Breitseiten der Materialbahn, das heißt bei der beschriebenen waagerechten Anordnung auf die Oberseite oder die Unterseite derselben, gestrahlt wird.

Die lichtemittierenden Bauteile beziehungsweise das linienförmige lichtemittierende Bauteil und entsprechend insbesondere auch die zugeordneten lichtempfangenden Sensoren sind vorteilhaft derart ausgeführt, dass das Lichtgitter, wenn es auf die Materialbahn trifft, auf diesem eine Linie, insbesondere gepunktete Linie, abbildet. Die Linie kann dabei senkrecht zu den Seitenkanten der Materialbahn verlaufen oder schräg hierzu. Insbesondere verläuft die Linie über die gesamte Breite der Materialbahn hinweg.

Bei Vorsehen einer Vielzahl von lichtemittierenden Bauteilen ist es vorzuziehen, dass jedem lichtempfangenden Sensor genau ein lichtemittierendes Bauteil zugeordnet wird. Das zugeordnete lichtemittierende Bauteil sendet einen einzigen, insbesondere gebündelten, Lichtstrahl zu dem lichtempfangenden Sensor. Beispielsweise kann jedes lichtemittierende Bauteil in Form einer einzigen Diode und jeder lichtempfangenden Sensor ebenfalls in Form einer einzigen Diode ausgeführt sein. Die lichtemittierenden Bauteile sind dann dementsprechend Senderdioden, und die lichtempfangenden Sensoren Empfängerdioden.

Insbesondere bei der zuvor beschriebenen Ausführung kann vorteilhaft der Vielzahl von lichtempfangenden Sensoren, insbesondere den Empfängerdioden, beispielsweise mittels einer zugeordneten Steuervorrichtung, ein Erkennungsmuster zugeordnet werden, das für jeden lichtempfangenden Sensor einen Lichtintensitätsgrenzwert vorgibt, der mit der tatsächlich von dem lichtempfangenden Sensor erfassten Lichtintensität verglichen wird. Dabei kommt nicht nur ein sogenannter Schwarz-/Weiß-Vergleich in Betracht, das heißt, es wird nicht unbedingt nur entschieden, ob Licht von dem zugeordneten lichtemittierenden Bauteil in dem lichtempfangenden Sensor ankommt, um so festzustellen, ob sich eine nicht transparente Materialbahn im Weg des Lichtes befindet und das Licht aus dem Sender abschirmt, sondern es ist auch möglich, die Lichtquelle des lichtemittierenden Bauteils derart in Abhängigkeit der Transparenz der Materialbahn einzustellen, dass stets Licht zumindest mit einer Restintensität den zugeordneten lichtempfangenden Sensor erreicht. Unter stets im Sinne der vorliegenden Erfindung ist entweder eine ununterbrochene lichtleitende Verbindung zwischen Sender und Empfänger gemeint oder eine im wesentlichen ununterbrochene Verbindung.

In Abhängigkeit des Vergleiches der tatsächlich erfassten Lichtintensität und der vorgegebenen Lichtintensität aus dem Erkennungsmuster kann festgestellt werden, ob die Materialbahn an dem dem lichtemittierenden Bauteil zugeordneten Zielpunkt die gewünschte Eigenschaft aufweist, das heißt, ob an diesem Zielpunkt ein Teil der Materialbahn vorhanden ist oder ob die Materialbahn in diesem Zielpunkt die gewünschte Beschaffenheit, insbesondere Dichte, aufweist und nicht bereits eine Rissbildung eingesetzt hat oder bevorsteht.

Beispielsweise ist es möglich, dass eine bereits unzulässig gedehnte Materialbahn in dem Zielpunkt mehr Licht durchlässt als dies gewünscht ist, was erfindungsgemäß durch Vergleich mit dem Erkennungsmuster festgestellt werden kann. Durch Vorsehen eines Lichtgitters können auch einzelne Löcher beziehungsweise Schwachstellen in der Materialbahn erkannt werden, die nicht oder noch nicht über der gesamten Breite der Materialbahn vorhanden sind.

Insbesondere wird in Abhängigkeit des Vergleichs ein Warnhinweis ausgegeben oder Sicherheitseingriffe, wie beispielsweise das Betätigen wenigstens einer Bahnabschlagvorrichtung, das Stoppen der Farbzufuhr, das Abschwenken beziehungsweise Öffnen von Rakelbalken und/oder Walzennips oder das Drosseln von Trocknerleistung eingeleitet. Allgemein besteht die Möglichkeit, um Schäden von Mensch und Maschine abzuhalten, der Maschine zugeordnete Aggregate, wie beispielsweise Trockner- oder Streicheinheiten, aus einer Arbeitsposition in eine Warteposition zu bringen, beispielsweise durch Abschwenken oder Verschieben, und/oder in ihrer Leistung zu vermindern oder abzuschalten, und die der Maschine zugeführten Medien, wie beispielsweise Wasser, Dampf oder Farbe, in ihrer Menge zu reduzieren oder abzustellen. Selbstverständlich sind auch andere Maßnahmen denkbar.

Besonders vorteilhaft ist eine Steuervorrichtung vorgesehen, die es ermöglicht, beispielsweise in Abhängigkeit der Materialbahneigenschaften das Erkennungsmuster zu programmieren. Gemäß eines Programmierverfahrens wird ein Erkennungsmuster automatisch in Abhängigkeit von eingegebenen und insbesondere an einem Eingabeterminal abgefragten Materialbahneigenschaften erzeugt, insbesondere in Abhängigkeit des eingegebenen Flächengewichts und/oder des bestimmten Verwendungszweckes.

Die erfindungsgemäße Vorrichtung weist analog zu den Merkmalen des beschriebenen Verfahrens ein (einziges) lichtemittierendes Bauteil mit einer linienförmigen Lichtquelle oder eine Vielzahl von nebeneinander angeordneten lichtemittierenden Bauteilen auf. Das Bauteil beziehungsweise die Bauteile sind auf einer ersten Seite der Materialbahn angeordnet. Auf einer zweiten Seite, welche der ersten Seite gegenüberliegt, ist eine Vielzahl von lichtempfangenden Sensoren angeordnet, welche dem oder den lichtemittierenden Bauteilen derart zugeordnet sind, dass das Licht, das von dem oder den lichtemittierenden Bauteilen in Richtung der zugeordneten lichtempfangenden Sensoren ausgestrahlt wird, ein Lichtgitter ausbildet, dass sich in Querrichtung der entlang ihrer Längsrichtung transportieren Materialbahn über die gesamte Breite derselben erstreckt.

Insbesondere wenn die lichtemittierenden Bauteile und die lichtempfangenden Sensoren in Form einer Senderleiste und einer Empfängerleiste ausgeführt sind, wobei beide Leisten vorteilhaft einteilig ausgeführt sind, beispielsweise durch Integration beziehungsweise Montage der oben beschriebenen Dioden, kann das Lichtgitter in Form eines Lichtbandes konstanter Breite ausgeführt werden, welches vorteilhaft aus einer Vielzahl von parallelen, gebündelten Lichtstrahlen zusammengesetzt ist.

Das erfindungsgemäß ausgesendete Licht weist vorzugsweise eine Wellenlänge im sichtbaren Bereich auf, insbesondere zwischen 380 und 780 Nanometer.

Die Erfindung soll nachfolgend anhand eines Ausführungsbeispiels exemplarisch beschrieben werden.

### Es zeigen:

- Figur 1: eine schräg zu einer Materialbahn angeordnete erfindungsgemäße Vorrichtung;
- Figur 2: schematisch den möglichen Aufbau der lichtemittierenden Bauteile und lichtempfangenden Sensoren in Form einer Senderleiste und einer Empfängerleiste, wie sie beispielsweise bei der Ausführung in der Figur 1 eingesetzt werden können.

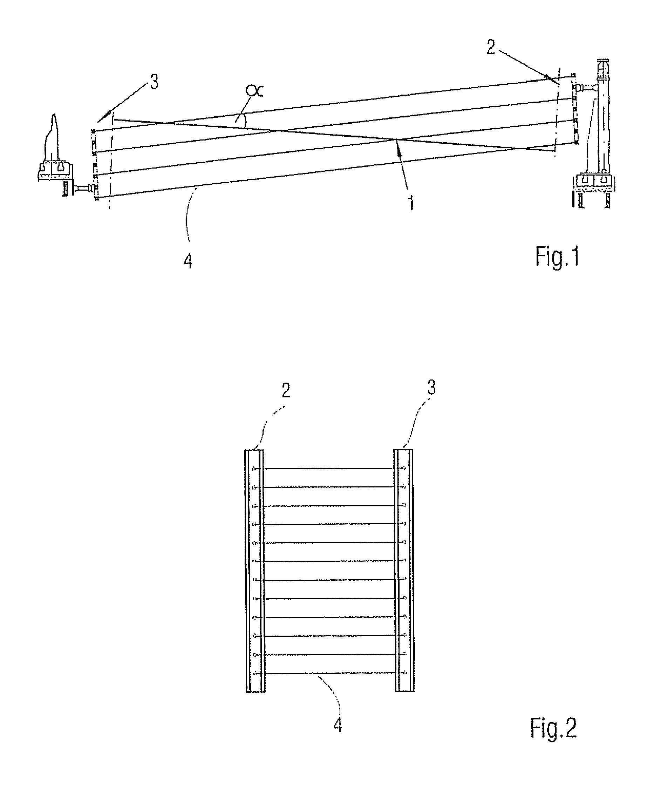

In der Figur 1 erkennt man eine Materialbahn 1, beispielsweise eine Papierbahn, die horizontal in einer entsprechenden Maschine, beispielsweise Papiermaschine (nur zwei Ständer rechts und links der Papierbahn 1 sind in Auszügen dargestellt), in ihrer Längsrichtung bewegt wird. Der Teil der Maschine kann beispielsweise eine Trockenpartie oder ein Teil einer Streichmaschine sein.

Mittels einer Vielzahl von lichtemittierenden Bauteilen 2 in Form einer Senderleiste, welche oberhalb der Materialbahn 1 angeordnet ist, wird ein Lichtgitter 4 schräg nach unten durch die Materialbahn 1 hindurch beziehungsweise durch Fehlstellen oder Abrissstellen derselben zu einer Vielzahl von lichtempfangenden Sendern 3 ausgestrahlt, wobei die letzteren ebenfalls in Form einer Schiene, nämlich einer Empfängerschiene, ausgeführt sind und unterhalb der Materialbahn 1 angeordnet sind.

Durch den gewählten spitzen Winkel α mit welchem das Lichtgitter 4 beziehungsweise die einzelnen parallel zueinander verlaufenden Lichtstrahlen, von denen hier schematisch vier Stück angedeutet sind, auf die Materialbahn 1, vorliegend auf deren Oberfläche, auftreffen, kann die Längserstreckung der Senderleiste und der Empfängerleiste im Verhältnis zu der Breite der Materialbahn 1 um ein Vielfaches kleiner ausgeführt sein. Wie man sieht, deckt das in einer seitlichen Draufsicht die Form eines Rechteckes aufweisende Lichtgitter 4 nahezu oder vollständig die gesamte Breite in Querrichtung der Materialbahn 1 ab.

Natürlich wäre es alternativ möglich, die lichtemittierenden Bauteile 2 beziehungsweise die Senderleiste waagerecht oberhalb oder unterhalb der Materialbahn 1 und dann vorteilhaft mit derselben Breite wie die Materialbahn 1 ausgeführt anzuordnen, und die lichtempfangenden Sensoren 3 beziehungsweise die Empfängerleiste in einer entsprechenden Ausführung auf der entgegengesetzt angeordneten Seite.

In der Figur 2 erkennt man die in Form von Leisten ausgeführten lichtemittierenden Bauteile 2 und lichtempfangenden Sensoren 3 in etwas größerem Detail. Wie man sieht, umfasst die Senderleiste eine Vielzahl von in regelmäßigen Abständen angeordnete und miteinander in Längsrichtung der Leiste fluchtende lichtemittierende Bauteile 2, beispielsweise Senderdioden. Die Empfängerleiste weist einen der Senderleiste entsprechenden Aufbau auf, nur dass anstelle jeder einzelnen Senderdiode eine Empfängerdiode als lichtempfangender Sender 3 vorgesehen ist.

Das Lichtgitter 4 wird demnach durch eine Vielzahl von parallelen, gebündelten Lichtstrahlen ausgebildet, die in ihrer Anzahl jeweils der Anzahl der lichtemittierenden Bauteile 2 beziehungsweise Senderdioden und lichtempfangenden Sensoren 3 beziehungsweise Empfängerdioden entsprechen.

## Patentansprüche

1. Verfahren zur Bahnabrissüberwachung in einer der Herstellung und/oder Behandlung einer Materialbahn (1), insbesondere einer Papier- oder Kartonbahn, dienenden Maschine, insbesondere Papier- oder Streichmaschine, in welcher die Materialbahn (1) mit einer vorgegebenen Geschwindigkeit in Längsrichtung entlang einer vorgegebenen Wegstrecke geführt wird; wobei ein Bahnabriss mittels eines lichtemittierenden Bauteils (2) und eines lichtempfangenden Sensors (3) erfasst wird, indem Licht von dem lichtemittierenden Bauteil (2) in Richtung der Materialbahn (1) zu einem vorgegebenen Zielpunkt gestrahlt wird, in Abhängigkeit des Vorhandenseins der Materialbahn in dem Zielpunkt reflektiert und/oder durchgelassen und/oder zu dem lichtempfangenden Sensor (3) geleitet wird; **dadurch gekennzeichnet, dass** das Licht derart von einer Vielzahl von nebeneinander auf einer ersten Seite der Materialbahn (1) angeordneten lichtemittierenden Bauteilen (2) oder von einem lichtemittierenden Bauteil (2) mit einer linienförmigen Lichtquelle auf der ersten Seite in Richtung einer zugeordneten Vielzahl von nebeneinander angeordneten lichtempfangenden Sensoren (3) auf einer zweiten, der ersten Seite entgegengesetzt angeordneten Seite der Materialbahn (1) gestrahlt wird, dass ein Lichtgitter (4) ausgebildet wird, welches sich in Querrichtung der Materialbahn (1) über die gesamte Breite derselben erstreckt.

2. Verfahren gemäß Anspruch 1, **dadurch gekennzeichnet, dass** das Lichtgitter (4) in einem spitzen Winkel α auf eine der beiden Breitseiten der Materialbahn (1) gestrahlt wird.

3. Verfahren gemäß einem der Ansprüche 1 oder 2, **dadurch gekennzeichnet, dass** das Lichtgitter (4) derart ausgestrahlt wird, dass es auf einer nicht abgerissenen Materialbahn (1) eine Linie abbildet, die senkrecht zu den Seitenkanten der Materialbahn (1) und über deren gesamte Breite verläuft.

4. Verfahren gemäß einem der Ansprüche 1 bis 3, **dadurch gekennzeichnet, dass** jedem lichtempfangenden Sensor (3) genau ein lichtemittierendes Bauteil (2) zugeordnet wird, welches einen Lichtstrahl, insbesondere gebündelten Lichtstrahl, in Richtung des lichtempfangenden Sensors (3) ausstrahlt.

5. Verfahren gemäß einem der Ansprüche 1 bis 4, **dadurch gekennzeichnet, dass** der Vielzahl von lichtempfangenden Sensoren (3) ein Erkennungsmuster zugeordnet wird, das für jeden lichtempfangenden Sensor (3) einen Lichtintensitätsgrenzwert vorgibt, der mit der tatsächlich von dem lichtempfangenden Sensor (3) erfassten Lichtintensität verglichen wird, und in Abhängigkeit dieses Vergleichs Warnmeldungen ausgegeben werden und/oder Sicherheitseingriffe an der Maschine vorgenommen werden.

6. Verfahren gemäß Anspruch 5, **dadurch gekennzeichnet, dass** eine Steuervorrichtung vorgesehen wird, mittels welcher das Erkennungsmuster, insbesondere in Abhängigkeit der Materialbahneigenschaften, programmierbar ist.

7. Verfahren gemäß Anspruch 6, **dadurch gekennzeichnet, dass** die Erzeugung eines Erkennungsmuster automatisch in Abhängigkeit von eingegebenen Materialbahneigenschaften, insbesondere des Flächengewichtes und/oder des Verwendungszweckes der Materialbahn (1), erfolgt.

8. Verfahren gemäß einem der Ansprüche 1 bis 7, **dadurch gekennzeichnet, dass** die Intensität des von dem oder den lichtemittierenden Bauteilen (2) ausgestrahlten Lichtes in Abhängigkeit der Lichtdurchlässigkeit der Materialbahn (1) derart eingestellt wird, dass die zugeordneten lichtempfangenden Sensoren (3) stets oder im wesentlichen stets das ausgestrahlte Licht empfangen, so dass die Intensität des empfangenen Lichtes bei vorhandener Materialbahn (1) in dem jeweiligen Zielpunkt, welcher dem jeweiligen lichtemittierenden Bauteil (2) zugeordnet ist, und/oder in Abhängigkeit der Anzahl der Lichtdurchgänge durch die Materialbahn (1) und/oder der Dichte der Materialbahn (1) in dem zugeordneten Sensor (3) geringer ist als bei nicht vorhandener Materialbahn (1).

9. Vorrichtung zur Bahnabrissüberwachung in einer der Herstellung und/oder Behandlung einer Materialbahn (1), insbesondere einer Papier- oder Kartonbahn, dienenden Maschine, insbesondere Papier- oder Streichmaschine, in welcher die Materialbahn (1) mit einer vorgegebenen Geschwindigkeit in Längsrichtung entlang einer vorgegebenen Wegstrecke geführt wird; mit wenigstens einem lichtemittierenden Bauteil (2); mit wenigstens einem lichtempfangenden Sensor (3), welcher dem lichtemittierenden Bauteil (2) derart gegenüberstehend zugeordnet ist, dass das Licht von dem lichtemittierenden Bauteil (2), das in Richtung eines vorgegebenen Zielpunktes gestrahlt wird, in Abhängigkeit des Vorhandenseins der Materialbahn (1) in dem Zielpunkt reflektiert und/oder durchgelassen und/oder zu dem lichtempfangenden Sensor (3) geleitet wird; **dadurch gekennzeichnet, dass** das lichtemittierende Bauteil (2) eine linienförmige Lichtquelle auf einer ersten Seite der Materialbahn (1) aufweist oder auf der ersten Seite eine Vielzahl von lichtemittierenden Bauteilen (2) nebeneinander angeordnet sind; und auf einer zweiten Seite der Materialbahn (1), welche entgegengesetzt zu der ersten Seite angeordnet ist, eine Vielzahl von lichtempfangenden Sensoren (3) vorgesehen sind, welche dem oder den lichtemittierenden Bauteilen (2) derart zugeordnet sind, dass das Licht, das von dem oder den lichtemittierenden Bauteilen (2) in Richtung der zugeordneten lichtempfangenden Sensoren (3) ausgestrahlt wird, ein Lichtgitter (4) ausbildet, das sich in Querrichtung der Materialbahn (1) über deren gesamte Breite erstreckt.

10. Vorrichtung gemäß Anspruch 9, **dadurch gekennzeichnet, dass** das oder die lichtemittierenden Bauteile (2) und die lichtempfangenden Sensoren (3) in Form einer, insbesondere einteiligen Senderleiste und einer, insbesondere einteiligen Empfängerleiste ausgeführt sind, welche sich auf entgegengesetzten Seiten der Materialbahn (1) derart gegenüberstehen, dass eine Vielzahl von parallelen, insbesondere gebündelten Lichtstrahlen in Form eines gitterförmigen Lichtbandes konstanter Breite von der Senderleiste in Richtung der Empfängerleiste ausgestrahlt wird.

11. Vorrichtung gemäß einem der Ansprüche 9 oder 10, **dadurch gekennzeichnet, dass** eine Steuervorrichtung vorgesehen ist, welche derart eingerichtet ist und mit dem oder den lichtemittierenden Bauteilen (2) und den lichtempfangenden Sensoren (3) verschaltet ist, dass sie ein Verfahren gemäß einem der Ansprüche 1 bis 8 steuert.

12. Maschine, insbesondere Papiermaschine oder Streichmaschine, die eine Materialbahn (1), insbesondere Papierbahn, in Längsrichtung derselben entlang einer vorgegebenen Wegstrecke transportiert, mit einer Vorrichtung gemäß einem der Ansprüche 9 bis 11, **dadurch gekennzeichnet, dass** das oder die lichtemittierenden Bauteile (2) und die lichtempfangenden Sensoren (3) derart schrägstehend gegenüber der Oberfläche der Materialbahn (1) angeordnet sind, dass das Lichtgitter (4) in einem spitzen Winkel α auf eine der beiden Breitseiten der Materialbahn (1) gestrahlt wird.
